# EUROPÄISCHE PATENTANMELDUNG

(11) **EP 1 408 313 A2**
(43) Veröffentlichungstag der Anmeldung: **14.04.2004**
(21) Anmeldenummer: 03017132.6
(22) Anmeldetag: 29.07.2003
(51) Int. Cl.: G01F 1/684, G01F 1/708

(54) **Thermischer Flusssensor**

(30) Priorität: 07.10.2002 DE 10246683
(71) Anmelder: Gottlieb Weinmann Geräte für Medizin und Arbeitsschutz GmbH + Co., 22525 Hamburg (DE)
(72) Erfinder: Hicken, Stefan, 25451 Quickborn (DE); Pulla, Matthias, 22529 Hamburg (DE)
(74) Vertreter: Klickow, Hans-Henning, Dr.-Ing.

(57) **Zusammenfassung**

Die Vorrichtung dient zur Messung von fluidischen Strömungen und weist mindestens einen Strömungssensor auf, der von einem Tragelement gehaltert ist. Das Tragelement ist gemeinsam mit dem Strömungssensor im Bereich eines Gehäuseteiles fixierbar. Der Strömungssensor ragt ausgehend vom Tragelement in einen Strömungsraum des Gehäuseteiles hinein.

## Beschreibung

Die Erfindung betrifft eine Vorrichtung zur Messung von fluidischen Strömungen, die mindestens einen Strömungssensor aufweist, der von einem Tragelement gehaltert ist.

Derartige Vorrichtungen sind in unterschiedlichen Ausführungsformen bekannt. In der DE-OS 196 47 350 wird beispielsweise eine Vorrichtung zur Strömungsmessung im Bereich von Gasen beschrieben, mit deren Hilfe ein Volumendurchfluß erfaßt werden kann. Die Messung erfolgt unter Verwendung eines Hitzdrahtes, der ein thermisches Signal erzeugt und eines in Strömungsrichtung nachfolgend angeordneten Sensors, der das Wärmesignal erfaßt. Ein Rückschluß über den vorliegenden Volumenfluß wird aus der ermittelten Laufzeit des Signals vom Hitzdraht bis zum Sensor gewonnen.

Die bislang bekannten Vorrichtungen sind noch nicht in optimaler Weise dafür geeignet, sowohl die Anforderungen an einen kompakten Aufbau, eine einfache Fertigung, eine zuverlässige Betriebsweise sowie an eine für den betreffenden Patienten angenehme Anordnung in Kombination miteinander zu erfüllen.

Aufgabe der vorliegenden Erfindung ist es daher, eine Vorrichtung der einleitend genannten Art derart zu konstruieren, daß eine kompakte sowie einfach zu fertigende und zu benutzende Meßeinrichtung bereitgestellt wird.

Diese Aufgabe wird erfindungsgemäß dadurch gelöst, daß das Tragelement gemeinsam mit dem Strömungssensor im Bereich eines Gehäuseteiles fixierbar ist und daß der Strömungssensor ausgehend vom Tragelement in einen Strömungsraum des Gehäuseteiles hineinragt.

Die Anordnung des Strömungssensors im Bereich des Tragelementes und die gemeinsame Fixierbarkeit im Bereich des Gehäuseteiles ermöglichen es, eine sehr kompakte Anordnung bereitzustellen und insbesondere im Bereich des Gehäuseteiles auch weitere Anschlüsse vorzusehen, um zusätzliche Parameter zu messen. Die Vorrichtung kommt deshalb mit wenigen Adaptoren und Verbindungen aus. Durch die Anordnung des vom Tragelement fixierten Sensors derart, daß dieser in den Innenraum des Gehäuseteiles hineinragt, wird ein inniger Kontakt mit der fluidischen Strömung gewährleistet, so daß der zu erfassende Strömungsparameter, beispielsweise ein jeweiliger Volumenfluß, mit hoher Genauigkeit erfaßt werden kann.
Der Strömungssensor mit Tragelement ist für unterschiedliche Anwendungen geeignet. Neben einem Einsatz bei Beatmungsgeräten für die Heimbeatmung oder zur Verwendung in Schlaflaboren kann eine Verwendung auch im Zusammenhang mit Anästhesiegeräten erfolgen.

Zu einer kompakten und stabilen Ausführungsform trägt es bei, daß das Tragelement mindestens bereichsweise plattenartig ausgebildet ist.

Eine verdrehsichere Anordnung wird insbesondere dadurch unterstützt, daß das Tragelement mindestens zwei Ausrichtelemente zur Positionierung im Bereich des Gehäuseteiles aufweist. Ebenfalls ist an die Verwendung einer oder mehrerer Rastnasen gedacht.

Zur Anpassung an rohrförmige oder schlauchförmige Leitungen für das Fluid wird vorgeschlagen, daß mindestens eines der Ausrichtelemente plattenartig ausgebildet und quer zu einer Längsachse des Gehäuseteiles angeordnet ist.

Zu einer kompakten und stabilen Ausführungsform trägt es ebenfalls bei, daß das Gehäuseteil mindestens bereichsweise rohrsegmentartig ausgebildet ist.

Zur Bereitstellung einer mechanisch belastbaren Verbindung zwischen dem Tragelement und dem Gehäuseteil wird vorgeschlagen, daß das Gehäuseteil mindestens eine Führung für das Ausrichtelement aufweist.

Eine bevorzugte Anwendung besteht darin, daß das Gehäuseteil mindestens bereichsweise ein Patientenventil ausbildet oder an ein Patientenventil ansetzbar ist.

Zur Unterstützung einer räumlich kompakten Erfassung mehrerer Meßparameter wird vorgeschlagen, daß das Gehäuseteil einen Anschlußstutzen für einen Meßschlauch aufweist. Hiermit kann beispielsweise der Anteil von Kohlendioxid im Atemgas ermittelt werden.

Zu einer zuverlässigen Sensorkonstruktion trägt es bei, daß der Strömungssensor mindestens vier Kontaktstifte aufweist.

Insbesondere ist daran gedacht, daß zwischen jeweils zwei Kontaktstiften ein Hitzdraht angeordnet ist.

Eine reproduzierbare Sensorherstellung wird dadurch unterstützt, daß der Hitzdraht durch Bonden mit den Kontaktstiften verbunden ist.

Eine zuverlässige elektrische Kontaktierung auch bei einem Einwirken äußerer Kräfte kann dadurch gewährleistet werden, daß das Tragelement im Bereich seiner dem Gehäuseteil abgewandten Ausdehnung mit einem Einfassungssteg versehen ist.

Ebenfalls trägt es zu einer zuverlässigen elektrischen Kontaktierung bei, daß auf den Einfassungssteg eine Anschlußkappe aufsetzbar ist. Hierdurch wird eine steckerartige Verbindung bereitgestellt.

Eine belastungsfähige Halterung von elektrischen Anschlußleitungen kann dadurch erfolgen, daß die Anschlußkappe mindestens ein Anschlußkabel haltert.

Zur Vergleichmäßigung der Strömung im Bereich des Strömungssensors wird vorgeschlagen, daß in mindestens eine der Führung ein Laminarisierungsgitter eingesetzt ist.

Zur Gewährleistung aussagefähiger Messungen wird vorgeschlagen, daß der Strömungssensor zur Durchführung von bidirektionalen Messungen ausgebildet ist.

Bidirektionale Messungen können in einfacher Weise dadurch realisiert werden, daß mindestens drei Hitzdrähte relativ zueinander parallel und beabstandet angeordnet sind.

Eine automatische Adaption der Meßeinrichtung an veränderliche Strömungen kann dadurch erfolgen, daß mindestens zwei Hitzdrähte wahlweise als Sender oder Empfänger ansteuerbar sind.

Eine erhöhte mechanische Stabilität kann dadurch erreicht werden, daß die Kontaktstifte von einem Sockel des Tragelementes gehaltert sind.

Eine Automatisierung einer Gesamteinrichtung wird dadurch unterstützt, daß das Patientenventil an eine Steuereinrichtung angeschlossen ist.

Insbesondere ist daran gedacht, daß die Steuerung das Patientenventil in Abhängigkeit von Meßdaten des Strömungssensors steuert.

Eine leichte Bauweise wird mit einer hohen mechanischen Stabilität und einer flexiblen Herstellbarkeit dadurch kombiniert, daß das Gehäuseteil mindestens bereichsweise aus einem Kunststoff ausgebildet ist.

Die oben aufgeführten Vorteile lassen sich noch dadurch erweitern, daß das Tragelement mindestens bereichsweise aus einem Kunststoff ausgebildet ist.

Ein typisches Meßverfahren wird dadurch definiert, daß der Strömungssensor an eine Steuerung zur Auswertung von Signallaufzeiten angeschlossen ist.

In den Zeichnungen sind Ausführungsbeispiele der Erfindung schematisch dargestellt. Es zeigen:
- Fig. 1: Eine Prinzipdarstellung einer Beatmungseinrichtung, bei der ein Strömungssensor von einem Gehäuseteil im Bereich eines Patientenventils angeordnet ist,
- Fig. 2: eine perspektivische vergrößerte Darstellung des Gehäuseteils mit Tragelement und Strömungssensor gemäß Fig. 1 in einem teilweise auseinandergenommenen Zustand,
- Fig. 3: einen Querschnitt durch das Tragelement mit gehaltertem Strömungssensor gemäß Schnittlinie III-III in Fig. 2,
- Fig. 4: einen Längsschnitt gemäß Schnittlinie IV-IV in Fig. 2,
- Fig. 5: einen Querschnitt gemäß Schnittlinie V-V in Fig. 3,
- Fig. 6: eine Darstellung eines Laminarisierungsgitters,
- Fig. 7: einen Längsschnitt gemäß Schnittlinie VII-VII in Fig. 4,
- Fig. 8: einen Längsschnitt gemäß Schnittlinie VIII-VIII in Fig. 3,
- Fig. 9: einen Querschnitt gemäß Schnittlinie IX-IX in Fig. 4,
- Fig. 10: einen Längsschnitt entsprechend der Darstellung in Fig. 4 nach einem Zusammenfügen des Tragelementes mit Strömungssensor und des Gehäuseteiles sowie nach einem Einsetzen von zwei Laminarisierungsgittern,
- Fig. 11: einen Querschnitt entsprechend Fig. 10 bei einer Blickrichtung gemäß Fig. 4 nach einem zusätzlichen Aufsetzen einer Anschlußkappe,
- Fig. 12: eine vergrößerte Darstellung der Einzelheit XII in Fig. 11,
- Fig. 13: eine perspektivische Darstellung eines Teiles des Strömungssensors mit sechs Kontaktstiften sowie drei relativ zueinander parallel verlaufenden und mit jeweils zwei Kontaktstiften durch Bonden verbundenen Hitzdrähten,
- Fig. 14: ein Notfallsbeatmungsgerät mit Anschlüssen für einen teilweise dargestellten Atemgasschlauch sowie einen ebenfalls teilweise dargestellten Druckmeßschlauch,
- Fig. 15: eine Beatmungsmaske mit zugeordnetem Patientenventil, das über den Atemgasschlauch mit dem Notfallbeatmungsgerät gemäß Fig. 14 verbindbar ist und
- Fig. 16: eine vergrößerte perspektivische Darstellung des Patientenventils mit Steckverbindung für den Strömungssensor.

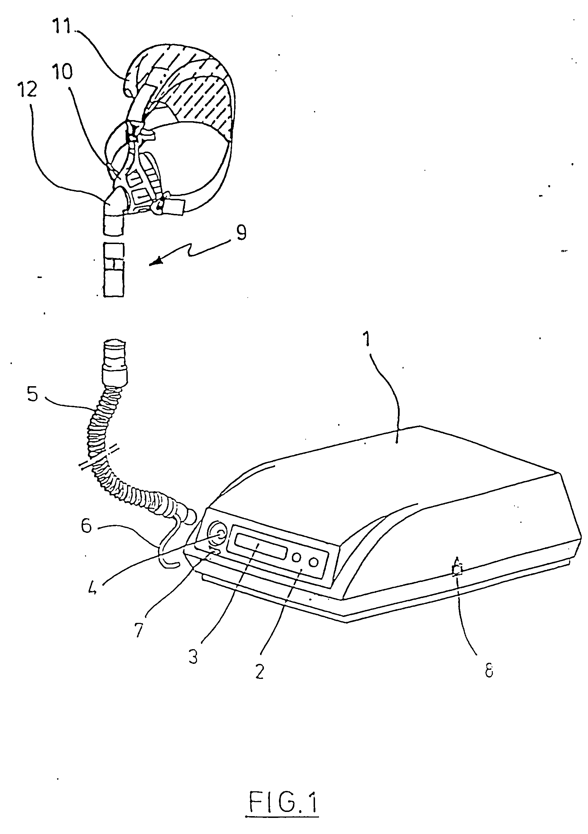

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein Verbindungsschlauch (5) angeschlossen. Entlang des Verbindungsschlauches (5) kann ein zusätzlicher Druckmeßschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

Im Bereich einer dem Gerätegehäuse (1) abgewandten Ausdehnung des Verbindungsschlauches (5) ist ein Patientenventil (9) zur Steuerung einer Gasströmungsrichtung in Abhängigkeit von Inspirations- und Exspirationsphasen angeordnet. Ebenfalls kann ein Ausatemelement in Form einer Luftableiteinrichtung verwendet werden.

Fig. 1 zeigt darüber hinaus eine Beatmungsmaske (10), die als Nasalmaske ausgebildet ist. Eine Fixierung im Bereich eines Kopfes eines Patienten kann über eine Kopfhaube (11) erfolgen. Im Bereich ihrer dem Verbindungsschlauch (5) zugewandten Ausdehnung weist die Beatmungsmaske (10) ein Kupplungselement (12) auf.

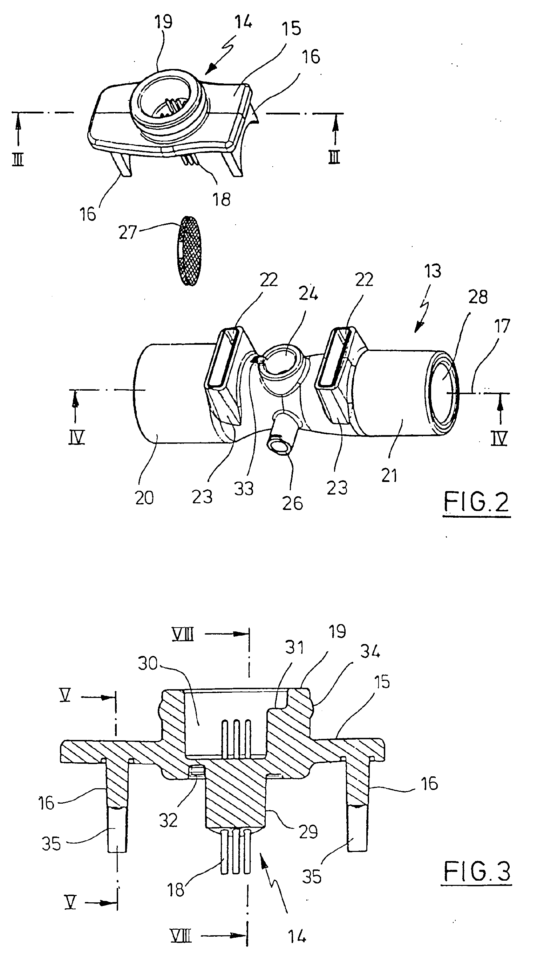

Fig. 2 zeigt ein Gehäuseteil (13) und den Strömungssensor (14) in einer perspektivischen und teilweise auseinander genommenen Darstellung. Das Gehäuseteil (13) kann beispielsweise als gemeinsames Bauteil integriert mit dem Patientenventil (9) ausgebildet oder zumindest in unmittelbarer Nähe des Patientenventils (9) angeordnet sein. Es ist zu erkennen, daß der Strömungssensor (14) von einem Tragelement (15) gehaltert ist, das bereichsweise einen plattenartigen Aufbau aufweist. Zur Verbindung mit dem Gehäuseteil (13) weist das Tragelement (15) zwei Ausrichtelemente (16) auf, die ebenfalls plattenartig ausgebildet sind. Die Ausrichtelemente (16) erstrecken sich im wesentlichen quer zu einer Längsachse (17) des Gehäuseteiles (13).

Im Bereich einer dem Gehäuseteil (13) zuwendbaren Ausdehnung des Tragelementes (15) ragen aus dem Tragelement (15) Kontaktstifte (18) heraus, die einen Teil des Strömungssensors (14) ausbilden. Die Kontaktstifte (18) ragen ebenfalls in einer dem Gehäuseteil (13) abgewandten Richtung aus dem Tragelement (15) heraus. Bei der in Fig. 2 dargestellten Ausführungsform sind die Kontaktstifte (18) im Bereich ihrer dem Gehäuseteil (13) abgewandten Ausdehnung von einem Einfassungssteg (19) umgeben.

Das Gehäuseteil (13) ist rohrartig ausgebildet und weist im Bereich seiner einander abgewandten Enden Verbindungsbereiche (20, 21) auf, die vorzugsweise zur Realisierung von Steckverbindungen ausgebildet sind. Das im wesentlichen rohrartig ausgebildete Gehäuseteil (13) weist hierzu im Verbindungsbereich (20) einen Innendurchmesser auf, der einem Außendurchmesser im Bereich des Verbindungsbereiches (21) entspricht. Hierdurch kann das Gehäuseteil (13) in einfacher Weise in Steckverbindungssysteme als Verlängerungsteil integriert werden.

Das Gehäuseteil (13) ist mit Führungen (22) für die Ausrichtelemente (16) versehen. Die Führungen (22) verlaufen im wesentlichen schlitzförmig und sind quer zur Längsachse (17) orientiert. Zur Vergrößerung eines jeweiligen Führungsbereiches erheben sich über eine Außenkontur des Gehäuseteiles (13) Führungseinfassungen (23). Die Führungseinfassungen stellen einen Kleberaum bereit. Zwischen den Führungseinfassungen (23) ist eine Sensoreinfassung (24) positioniert, die eine Sensorausnehmung (25) begrenzt. In die Sensorausnehmung (25) ist der vom Tragelement (15) positionierte Strömungssensor (14) einführbar.

Quer zur Längsachse (17) erstreckt sich ein Anschlußstutzen (26), auf den ein nicht dargestellter Schlauch aufgesteckt werden kann. Über den Anschlußstutzen (26) kann ein Teil des Strömungsvolumens zur Durchführung einer Messung des Gehaltes an Kohlendioxid abgezweigt werden. Ebenfalls ist alternativ oder ergänzend über den Anschlußstutzen (26) eine Druckmessung durchführbar. Hierzu wird der Druckmeßschlauch (6) auf den Anschlußstutzen (26) aufgesteckt.

Fig. 2 zeigt zusätzlich schematisch ein Laminarisierungsgitter (27), das in die Führung (22) einsetzbar ist. Grundsätzlich ist es möglich, in beide Führungen (22) jeweils ein Laminarisierungsgitter (27) einzusetzen. Das Gehäuseteil (13) begrenzt mit einer Innenkontur einen Strömungsraum (28), der seinerseits wenigstens abschnittsweise zylindrisch begrenzt ist, wobei in Richtung der Längsachse (17) zylindrische Bereiche und Überleitungsbereiche mit variierenden Innendurchmessern angeordnet sind. Die Ausrichtelemente (16) des Tragelementes (15) weisen einen Konturverlauf auf, der an den Konturverlauf des Strömungsraumes (28) angepaßt ist. Hierdurch werden Querschnittverengungen und Verwirbelungen des strömenden Fluids vermieden.

Fig. 3 zeigt in einer Querschnittdarstellung die Anordnung des Strömungssensors (14) im Bereich des Tragelementes (15) in einer stärker detaillierten Darstellung. Insbesondere ist zu erkennen, daß das Tragelement (15) mit einem Sockel (29) versehen ist, der die Kontaktstifte (18) haltert und in ihrer jeweiligen Positionierung stabilisiert. Ebenfalls ist zu erkennen, daß in einem vom Einfassungssteg (19) umschlossenen Kontaktraum (30) ein vom Einfassungssteg (19) ausgehender Positionierungsvorsprung (31) angeordnet ist. Der Positionierungsvorsprung (31) dient dazu, bei einer Verwendung von Steckverbindungen zur elektrischen Kontaktierung der Kontaktstifte (18) eine definierte Zuordnung der jeweiligen Verbindungen sicherzustellen. Im Bereich des Tragelementes (15) ist ein Profil (32) angeordnet, das mit einem Gegenprofil (33) im Bereich des Gehäuseteiles (13) zusammenwirkt. Auch das Profil (32) und das Gegenprofil (33) stellen einen definierten Zusammenbau des Tragelementes (15) und des Gehäuseteiles (13) sicher. Beim dargestellten Ausführungsbeispiel ist das Profil (32) als eine Vertiefung und das Gegenprofil (33) als ein angepaßt konturierter Vorsprung realisiert.

Zu erkennen ist in Fig. 3 auch ein Außenprofil (34) des Einfassungssteges (19), das als gerundet konturierter Vorsprung realisiert ist und außenseitig entlang des Einfassungssteges (19) verläuft. Ebenfalls sind gerundet verlaufende Innenkonturen der Ausrichtelemente (16) im Querschnitt zu erkennen.

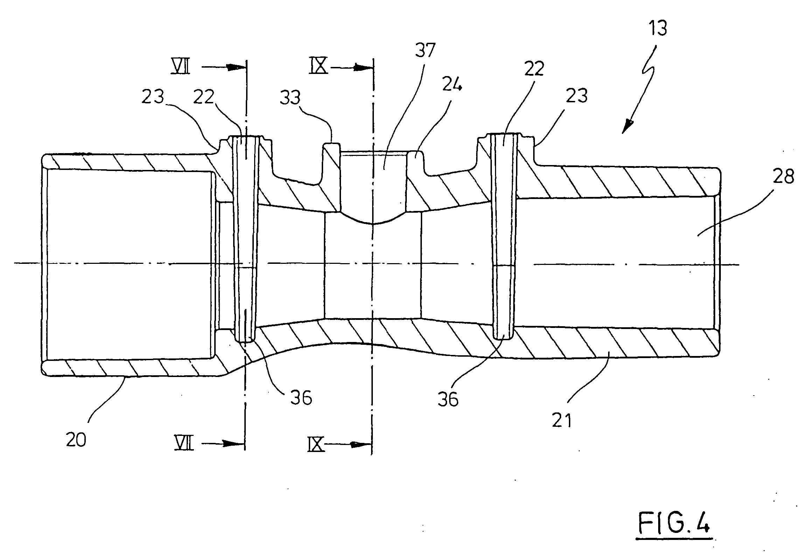

Fig. 4 veranschaulicht in einer Längsschnittdarstellung den Konturverlauf des Strömungsraumes (28). Ebenfalls ist zu erkennen, daß im Bereich der Wandung des Gehäuseteiles (13) innenseitig als Verlängerung der Führungen (22) Vertiefungen (36) angeordnet sind. Die Vertiefungen (36) stabilisieren die Anordnung der Laminarisierungsgitter (27) im Bereich des Gehäuseteiles (13). Ebenfalls ist zu erkennen, daß die Sensoreinfassung (24) eine Sensoröffnung (37) des Gehäuseteiles (13) umgibt, in die der Strömungssensor (14) mit seinem Sockel (29) einführbar ist.

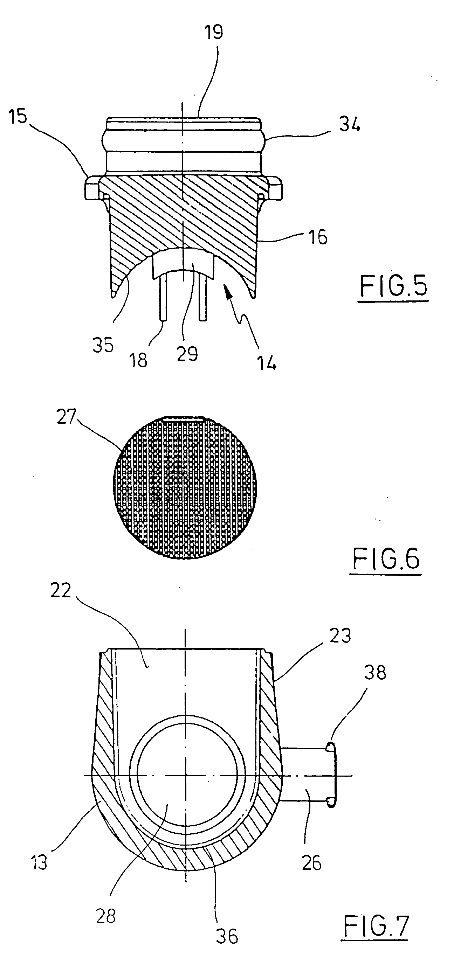

Fig. 5 veranschaulicht, daß das Tragelement (15) in einer Umgebung des Einfassungssteges (19) breiter ausgebildet ist als in einer Umgebung der Ausrichtelemente (16).

Aus Fig. 6 ist zu erkennen, daß das Laminarisierungsgitter (27) vorzugsweise kreisförmig ausgebildet und mit einer gleichmäßigen Gitterstruktur versehen ist.

Fig. 7 veranschaulicht die schlitzförmige Struktur der Führung (22). Ebenfalls ist zu erkennen, daß der Anschlußstutzen (16) mit einem Verbindungsprofil (28) versehen ist, um eine sichere Fixierung eines Anschlußschlauches auch bei einer Einwirkung von Zugkräften zu unterstützen. Das Verbindungsprofil (38) kann als Außenwulst ausgebildet sein, es ist aber auch möglich, beispielsweise eine gewindeartige Struktur zu realisieren.

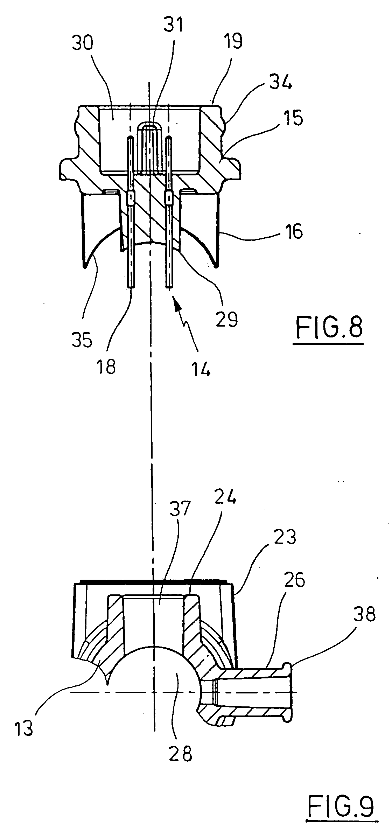

Fig. 8 veranschaulicht zum einen die Gestaltung des Positionierungsvorsprunges (31), darüber hinaus ist zu erkennen, daß die Kontaktstifte (18) im Bereich des Sockels (29) mit Profilverdickungen (39) versehen sind. Die Profilverdickungen (39) verhindern eine Längsverschiebung der Kontaktstifte (18) innerhalb des Sockels (29). Eine Herstellung des Tragelementes (15) mit Kontaktstiften (18) kann beispielsweise derart erfolgen, daß die Kontaktstifte (18) innerhalb eines Spritzgußwerkzeuges positioniert und anschließend mit einem Spritzgußmaterial umspritzt werden. Nach einer ausreichenden Materialverfestigung und Entformung wird das Tragelement (15) mit dauerhaft fixierten Kontaktstiften (18) bereitgestellt.

Der Querschnitt in Fig. 9 veranschaulicht die Einmündung der Sensoröffnung (37) und eines Innenraumes des Anschlußstutzens (26) in den Strömungsraum (28). Insbesondere ist zu erkennen, daß die Sensoröffnung (37) und der Innenraum des Anschlußstutzens (26) relativ eng benachbart in den Strömungsraum (28) einmünden.

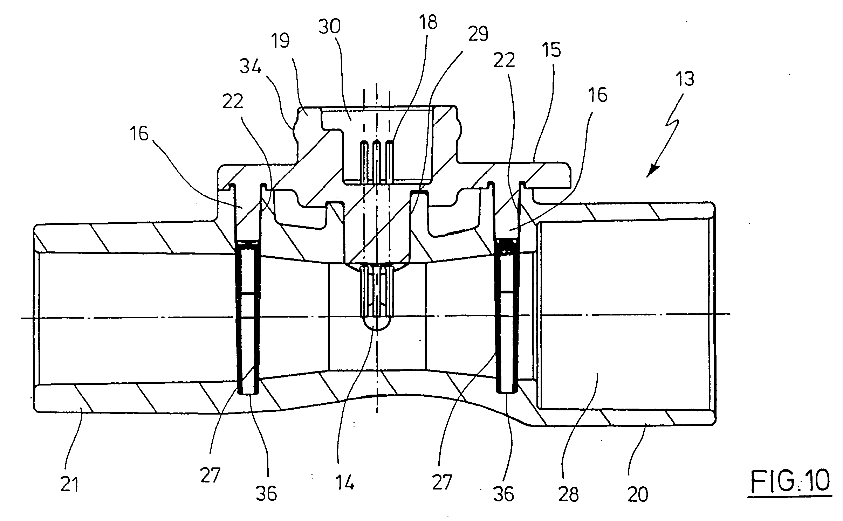

Fig. 10 zeigt das Gehäuseteil (13) gemäß Fig. 4 nach einem Einsetzen des Tragelementes (15) bei einer Blickrichtung aus der Zeichnungsebene von Fig. 4 heraus. In die Führungen (22) sind auch bereits die Laminarisierungsgitter (27) eingesetzt. Zu erkennen ist insbesondere, daß die Laminarisierungsgitter (27) durch die Vertiefungen (36) des Gehäuseteiles (13) ausgerichtet sind.

Die vom Sockel (29) des Tragelementes (15) gehalterten Kontaktstifte (18) ragen in den Strömungsraum (28) hinein, um einen guten Kontakt zwischen dem Strömungssensor (14) und dem innerhalb des Strömungsraumes (28) strömenden Fluids zu gewährleisten.

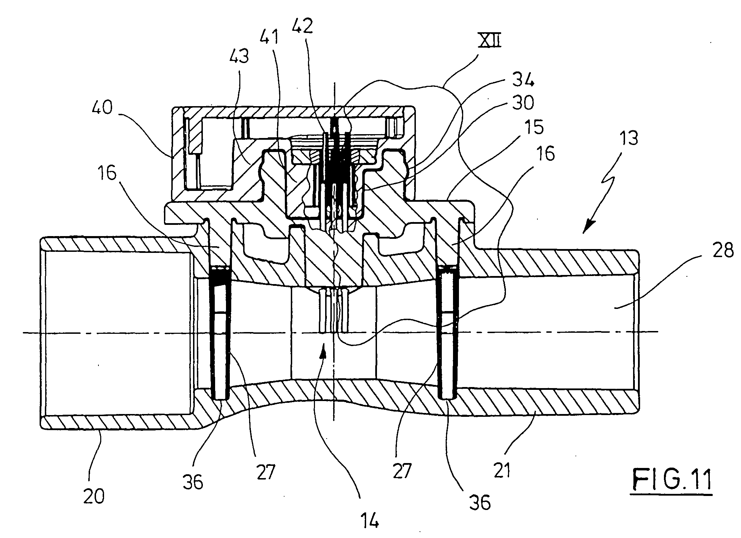

Fig. 11 zeigt das Gehäuseteil (13) gemäß Fig. 4 nach einem zusammenfügen mit dem Tragelement (15) und einem zusätzlichen Aufsetzen einer Anschlußkappe (40) auf das Tragelement (15).

In Fig. 11 weist das Tragelement (15) wieder eine mit Fig. 4 übereinstimmende räumliche Orientierung auf. Die Anschlußkappe (40) wird mit einem Kontaktsockel (41) in den Kontaktraum (30) des Tragelementes (15) eingeführt und weist Kontaktbuchsen (42) zur Herstellung einer elektrischen Verbindung mit den Kontaktstiften (18) auf. Zur Sicherstellung einer zuverlässigen Positionierung der Anschlußkappe (40) greift diese mit einem Innenprofil (43) in das Außenprofil (34) des Tragelementes (15) ein. Es wird hierdurch eine Verbindung ähnlich zu einem Schnappverschluß bereitgestellt, bei dem eine definierte Druckkraft zur Herstellung einer formschlüssigen Verbindung und eine definierte Zugkraft zur Aufhebung dieser Verbindung bereitgestellt werden müssen.

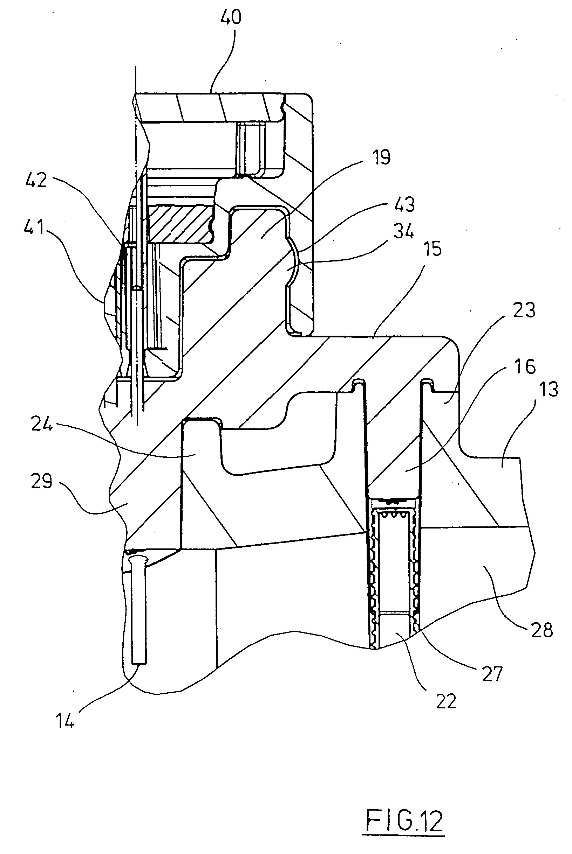

Zur Veranschaulichung einer Kombination der einzelnen Bauelemente miteinander zeigt Fig. 12 eine vergrößerte Darstellung der Einzelheit XII aus Fig. 11. Fig. 12 veranschaulicht insbesondere noch einmal die Halterung des Trageelementes (15) durch das Gehäuseteil (13), die Positionierung des Strömungssensors (14) im Bereich des Tragelementes (15) und die Anordnung der Anschlußkappe (40) auf dem Tragelement (15).

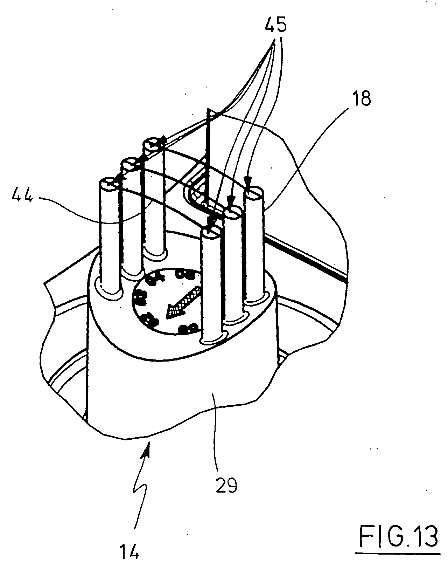

Fig. 13 zeigt in einer perspektivischen Darstellung den Strömungssensor (14). Es ist insbesondere zu erkennen, daß zwischen jeweils zwei Kontaktstiften (18) ein Hitzdraht (44) angeordnet ist. Elektrische Verbindungen (45) zwischen den Hitzdrähten (44) und den jeweils zugeordneten Kontaktstiften (18) können durch ein Bonden erzeugt werden. Die Hitzdrähte (44) verlaufen relativ zueinander parallel und nach einer Anordnung des Strömungssensors (14) im Gehäuseteil (13) quer zur Längsachse (17).

Die Hitzdrähte (44) werden über die Anschlußkappe (40) an eine Steuerung angeschlossen, die sowohl durch einen oder mehrere Hitzdrähte (44) Heizströme schickt und auch die erforderlichen Messungen durchführt. Durch die gewählte Anordnung ist eine bidirektionale Messung möglich. Insbesondere können die Hitzdrähte (44) auch wahlweise als Signalgeber und als Signalempfänger geschaltet werden. Hierdurch ist es insbesondere möglich, die Länge der Meßstrecke zu variieren. Wird beispielsweise der eine außen liegende Hitzdraht (44) als Signalgeber und der andere außen liegende Hitzdraht (44) als Signalempfänger geschaltet, so ergibt sich eine lange Meßstrecke, die bei schnell strömenden Fluiden vorteilhaft ist. Es können aber auch zwei benachbarte Hitzdrähte (44) als Sender bzw. als Empfänger geschaltet werden, so daß eine kurze Meßstrecke vorliegt, die bei langsam strömenden Fluiden vorteilhaft ist.

Zur Durchführung einer Messung wird an den in Strömungsrichtung ersten Hitzdraht (44) ein in der Frequenz modulierbarer Strom angelegt, durch den ein sinusförmiges Temperatursignal an das strömende Medium abgegeben wird. Das Temperatursignal wird vom zweiten Hitzdraht (44) aufgenommen und verändert dessen Temperatur und bei Verwendung von Hitzdrähten (44) mit temperaturabhängigen Widerständen auch dessen elektrischen Widerstand.

Bei einem vorgegebenen Stromfluß durch den zweiten Hitzdraht (44) führt die Änderung des elektrischen Widerstandes zu einer Änderung der am zweiten Hitzdraht anliegenden Spannung. In Abhängigkeit vom Strömungsvolumen tritt eine Phasenverschiebung zwischen dem durch den ersten Hitzdraht (44) fließenden Strom und der am zweiten Hitzdraht (44) anliegenden Spannung auf. Aus dieser Phasenverschiebung kann die Laufzeit des Temperatursignals zwischen den beiden Hitzdrähten (44) und damit die Strömungsgeschwindigkeit des Fluids bestimmt werden. Die Laufzeit sowie die Querschnittfläche des Strömungsraumes ergeben den jeweiligen Volumenstrom. Über die Auswertungseinrichtung wird die entsprechende Information über den Volumenstrom zeitlich integriert, so daß Informationen über das jeweilige Inspirationsvolumen bzw. Exspirationsvolumen vorliegen. Die jeweilige Messung kann unabhängig von der jeweiligen Signalamplitude durchgeführt werden, da lediglich die Laufzeit in das Meßergebnis eingeht.

Stoffeigenschaften des Mediums und insbesondere unterschiedliche Wärmekapazitäten des Mediums in Abhängigkeit vom konkret anliegenden Druck, von der aktuellen Feuchtigkeit sowie von anderen Parametern gehen somit nicht in das Meßergebnis ein. Eine Kalibrierung der Einrichtung für unterschiedliche Medien oder unterschiedliche Anwendungsbedingungen entfällt somit.

Durch einen Vergleich zeitlich aufeinander folgender Meßwerte ist es möglich, Änderungen der Strömungsrichtung zu erfassen und bei Erkennung eines derartigen Betriebszustandes die Funktion von Sender und Empfänger zu vertauschen. Zur Durchführung der Messungen ist es stets erforderlich, daß in Strömungsrichtung zunächst der Sender und in Strömungsrichtung abwärts der Empfänger positioniert ist, da andernfalls kein Meßsignal detektierbar ist.

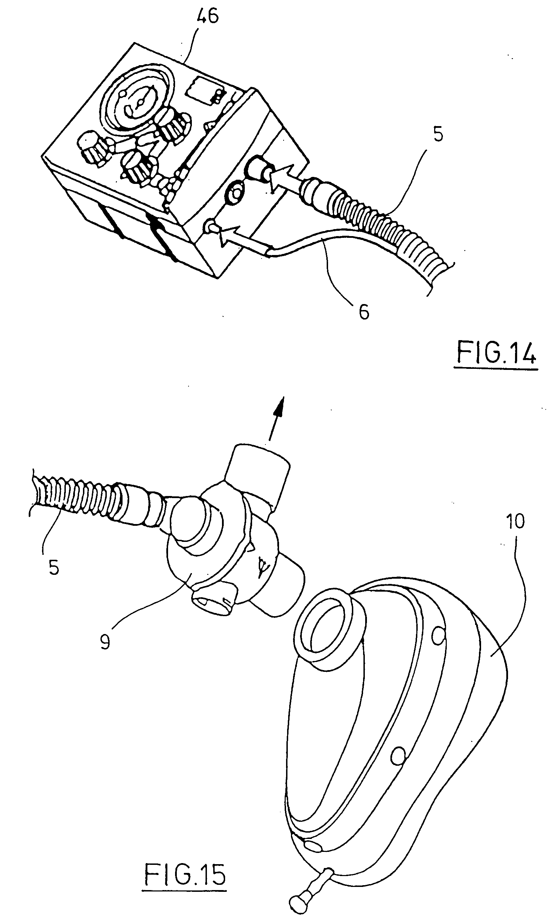

Fig. 14 zeigt ein Notfallbeatmungsgerät (46), das über den Verbindungsschlauch (5) mit dem in Fig. 15 dargestellten Patientenventil (9) verbindbar ist. Ebenfalls ist in Fig. 14 ein im wesentlichen parallel zum Verbindungsschlauch (5) verlaufender Druckmeßschlauch (6) zu erkennen.

Fig. 15 zeigt das Patientenventil (9), das mit der Beatmungsmaske (10) verbindbar ist. Ein Atemgasausgang (47) des Patientenventils (9) ist bei der gewählten Darstellung im wesentlichen in lotrechter Richtung nach oben orientiert.

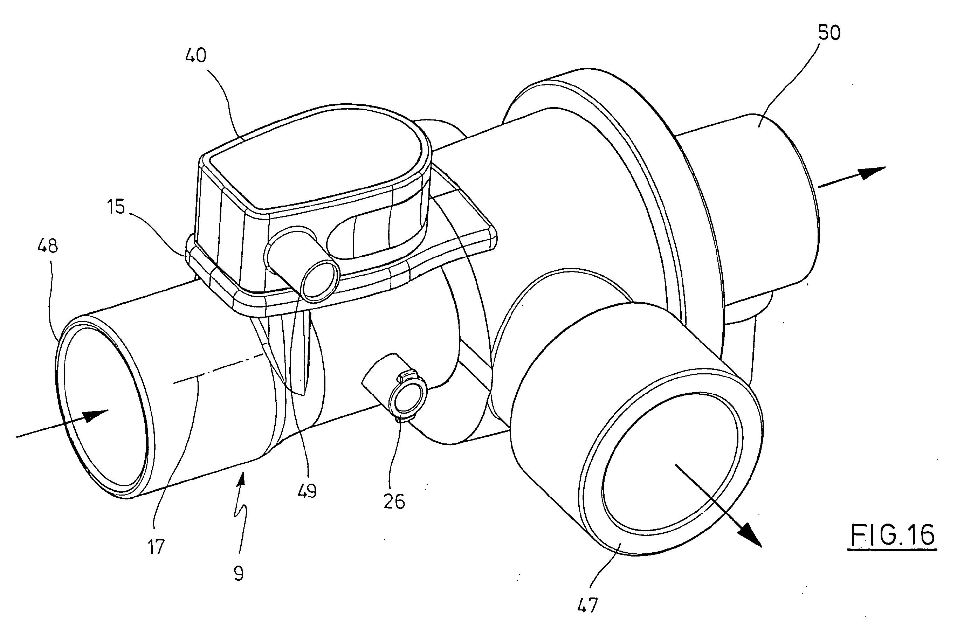

Fig. 16 zeigt das Patientenventil (9) mit Tragelementen (15) sowie aufgesetzter Anschlußkappe (40) in einer perspektivischen Darstellung. Der Verbindungsschlauch (5) wird im Bereich eines Atemgaseinganges (48) mit dem Patientenventil (9) verbunden. Der Anschlußstutzen (26) sowie eine stutzenartige Kabelführung (49) der Anschlußkappe (40) verlaufen im wesentlichen quer zur Längsachse (17). Die Längsachse (17) verläuft vom Atemgaseingang (48) in Richtung auf einen Maskenanschluß (50).

## Patentansprüche

1. Vorrichtung zur Messung von fluidischen Strömungen, die mindestens einen Strömungssensor aufweist, der von einem Tragelement gehaltert ist, **dadurch gekennzeichnet, daß** das Tragelement (15) gemeinsam mit dem Strömungssensor (14) im Bereich eines Gehäuseteiles (13) fixierbar ist und daß der Strömungssensor (14) ausgehend vom Tragelement (15) in einen Strömungsraum (28) des Gehäuseteiles (13) hineinragt.

2. Vorrichtung nach Anspruch 1, **dadurch gekennzeichnet, daß** das Tragelement (15) mindestens bereichsweise plattenartig ausgebildet ist.

3. Vorrichtung nach Anspruch 1 oder 2, **dadurch gekennzeichnet, daß** das Tragelement (15) mindestens zwei Ausrichtelemente (16) zur Positionierung im Bereich des Gehäuseteiles (13) aufweist.

4. Vorrichtung nach einem der Ansprüche 1 bis 3, **dadurch gekennzeichnet, daß** mindestens eines der Ausrichtelemente (16) plattenartig ausgebildet und quer zu einer Längsachse (17) des Gehäuseteiles (13) angeordnet ist.

5. Vorrichtung nach einem der Ansprüche 1 bis 4, **dadurch gekennzeichnet, daß** das Gehäuseteil (13) mindestens bereichsweise rohrsegmentartig ausgebildet ist.

6. Vorrichtung nach einem der Ansprüche 1 bis 5, **dadurch gekennzeichnet, daß** das Gehäuseteil (13) mindestens eine Führung (22) für das Ausrichtelement (16) aufweist.

7. Vorrichtung nach einem der Ansprüche 1 bis 6, **dadurch gekennzeichnet, daß** das Gehäuseteil (13) mindestens bereichsweise ein Patientenventil ausbildet.

8. Vorrichtung nach einem der Ansprüche 1 bis 7, **dadurch gekennzeichnet, daß** das Gehäuseteil (13) einen Anschlußstutzen (26) für einen Meßschlauch aufweist.

9. Vorrichtung nach einem der Ansprüche 1 bis 8, **dadurch gekennzeichnet, daß** der Strömungssensor (14) mindestens vier Kontaktstifte (18) aufweist.

10. Vorrichtung nach Anspruch 9, **dadurch gekennzeichnet, daß** zwischen jeweils zwei Kontaktstiften (18) ein Hitzdraht (44) angeordnet ist.

11. Vorrichtung nach Anspruch 9 oder 10, **dadurch gekennzeichnet, daß** der Hitzdraht (44) durch Bonden mit den Kontaktstiften (18) verbunden ist.

12. Vorrichtung nach einem der Ansprüche 1 bis 11, **dadurch gekennzeichnet, daß** das Tragelement (15) im Bereich seiner dem Gehäuseteil (13) abgewandten Ausdehnung mit einem Einfassungssteg (19) versehen ist.

13. Vorrichtung nach einem der Ansprüche 1 bis 12, **dadurch gekennzeichnet, daß** auf den Einfassungssteg (19) eine Anschlußkappe (40) aufsetzbar ist.

14. Vorrichtung nach einem der Ansprüche 1 bis 13, **dadurch gekennzeichnet, daß** die Anschlußkappe (40) mindestens ein Anschlußkabel haltert.

15. Vorrichtung nach einem der Ansprüche 1 bis 14, **dadurch gekennzeichnet, daß** in mindestens eine der Führungen (22) ein Laminarisierungsgitter (27) eingesetzt ist.

16. Vorrichtung nach einem der Ansprüche 1 bis 15, **dadurch gekennzeichnet, daß** der Strömungssensor (14) zur Durchführung von bidirektionalen Messungen ausgebildet ist.

17. Vorrichtung nach einem der Ansprüche 1 bis 16, **dadurch gekennzeichnet, daß** mindestens drei Hitzdrähte (44) relativ zueinander parallel und beabstandet angeordnet sind.

18. Vorrichtung nach einem der Ansprüche 1 bis 17, **dadurch gekennzeichnet, daß** mindestens zwei Hitzdrähte (44) wahlweise als Sender oder Empfänger ansteuerbar sind.

19. Vorrichtung nach einem der Ansprüche 1 bis 18, **dadurch gekennzeichnet, daß** die Kontaktstifte (18) von einem Sockel (29) des Tragelementes (15) gehaltert sind.

20. Vorrichtung nach einem der Ansprüche 1 bis 19, **dadurch gekennzeichnet, daß** das Patientenventil (9) an eine Steuereinrichtung angeschlossen ist.

21. Vorrichtung nach Anspruch 20, **dadurch gekennzeichnet, daß** die Steuerung das Patientenventil (9) in Abhängigkeit von Meßdaten des Strömungssensors (14) steuert.

22. Vorrichtung nach einem der Ansprüche 1 bis 21, **dadurch gekennzeichnet, daß** das Gehäuseteil (13) mindestens bereichsweise aus einem Kunststoff ausgebildet ist.

23. Vorrichtung nach einem der Ansprüche 1 bis 21, **dadurch gekennzeichnet, daß** das Tragelement (15) mindestens bereichsweise aus einem Kunststoff ausgebildet ist.

24. Vorrichtung nach einem der Ansprüche 1 bis 23, **dadurch gekennzeichnet, daß** der Strömungssensor (14) an eine Steuerung zur Auswertung von Signallaufzeiten angeschlossen ist.
